# EUROPEAN PATENT APPLICATION

(11) **EP 3 643 303 A1**
(43) Date of publication of application: **29.04.2020**
(21) Application number: 18382751.8
(22) Date of filing: 22.10.2018
(51) Int. Cl.: A61K 31/05, A61P 19/04

(54) **COMPOUNDS FOR USE IN THE TREATMENT OR PREVENTION OF FIBROTIC DISEASES; PHARMACEUTICAL, COSMETIC COMPOSITIONS AND USES THEREOF**

(71) Applicant: InnovativeHealth Group SL, 28049 Madrid (ES)
(72) Inventor: Collado rojas, Juan Antonio, 14004 Córdoba (ES); Millán Ortega, Estrella, 28822 Coslada Madrid (ES); Muñoz Blanco, Eduardo, 14004 Córdoba (ES)
(74) Representative: Manuel Illescas y Asociados, S.L.U.

(57) **Abstract**

Cannabidiol enantiomers for use in the treatment and/or prevention of fibrotic diseases and/or in the treatment and/or prevention of fibrotic symptoms of fibrotic diseases in an animal or human and pharmaceutical compositions thereof by topical administration. The invention also relates to cosmetic compositions comprising cannabidiol enantiomers and cosmetic use thereof for preventing keloids, increasing skin elasticity and/or reducing skin itching and/or reducing skin pain in a subject.

SEQUENCE LISTING

<110> INNOVATIVEHEALTH GROUP SL

<120> Compounds for use in the treatment or prevention of fibrotic diseases;
pharmaceutical, cosmetic compositions and uses thereof

<130> EP-07968

<160> 14

<170> BiSSAP 1.3.6

<210> 1
<211> 21
<212> DNA
<213> Artificial Sequence

<220>
<223> IL-6 forward primer

<400> 1
gaacaacgat gatgcacttg c                                                21

<210> 2
<211> 21
<212> DNA
<213> Artificial Sequence

<220>
<223> IL-6 reverse primer

<400> 2
tccaggtagc tatggtactc c                                                21

<210> 3
<211> 19
<212> DNA
<213> Artificial Sequence

<220>
<223> IL-13 forward primer

<400> 3
cctggctctt gcttgcctt                                                   19

<210> 4
<211> 21
<212> DNA
<213> Artificial Sequence

<220>
<223> IL-13 reverse primer

<400> 4
ggtcttgtgt gatgttgctc a                                                21

<210> 5
<211> 19
<212> DNA
<213> Artificial Sequence

<220>
<223> Ccl2 forward primer

<400> 5
gggcctgctg ttcacagtt                                                    19

<210> 6
<211> 19
<212> DNA
<213> Artificial Sequence

<220>
<223> Ccl2 reverse primer

<400> 6
ccagcctact cattgggat                                                    19

<210> 7
<211> 20
<212> DNA
<213> Artificial Sequence

<220>
<223> IL-1Î² forward primer

<400> 7
ctccacctca atggacagaa                                                   20

<210> 8
<211> 20
<212> DNA
<213> Artificial Sequence

<220>
<223> IL-1Î² reverse primer

<400> 8
gccgtctttc attacacagg                                                   20

<210> 9
<211> 20
<212> DNA
<213> Artificial Sequence

<220>
<223> TGFÎ² forward primer

<400> 9
tacagcaagg tccttgccct                                                    20

<210> 10
<211> 16
<212> DNA
<213> Artificial Sequence

<220>
<223> TGFÎ² reverse primer

<400> 10
gcagcacggt gacgcc                                                        16

<210> 11
<211> 20
<212> DNA
<213> Artificial Sequence

<220>
<223> IL-4 forward primer

<400> 11
ggtctcaacc cccagctagt                                                    20

<210> 12
<211> 23
<212> DNA
<213> Artificial Sequence

<220>
<223> IL-4 reverse primer

<400> 12
gccgatgatc tctctcaagt gat                                                23

<210> 13
<211> 22
<212> DNA
<213> Artificial Sequence

<220>
<223> GAPDH forward primer

<400> 13
tggcaaagtg gagattgttg cc                                                 22

<210> 14
<211> 21
<212> DNA
<213> Artificial Sequence

<220>

<223> GAPDH reverse primer

<400> 14
aagatggtga tgggcttccc g                                                21

## Description

### Technical Field

This invention is in the field of medicinal chemistry and relates to cannabidiol enantiomers and pharmaceutical compositions thereof to treat and/or to prevent fibrotic diseases by topical administration. The invention also relates to cosmetic compositions and cosmetic uses of cannabidiol enantiomers.

### Background Art

Plant-derived cannabinoids (phytocannabinoids) refer to nearly 200 lipid-soluble compounds found exclusively in the plant *Cannabis sativa,* and different plant varieties differ in their content of phytocannabinoids (Hanuš et al. Nat Prod Rep. 2016; 33(12):1357-1392). The two major cannabinoids, Δ⁹tetrahydrocannabinol (Δ⁹-THC) and cannabidiol (CBD) are derived from the common synthetic precursor cannabigerol, which is also abundant in the hemp varieties of Cannabis containing low levels of Δ⁹-THC.
Δ ⁹-THC is the main psychotropic cannabinoids in the plant and cannabis potency is primarily evaluated according to a sample's Δ⁹-THC concentration. Δ9-THC was initially identified by its ability to bind and activate the classical cannabinoid receptors CB₁ and CB₂. However, cannabinoids such as Δ⁹-THC and CBD are pleiotropic in nature and also activate other type of receptors such as transient receptor potential cation channels, ligand-gated ion channels, deorphanized G protein-coupled receptors and peroxisome proliferator activated receptors such as PPARγ. Thus, cannabinoids may exert their biological activities through CB₁/CB₂-dependent and -independent mechanisms, or a combination of both. Therefore, it is not surprising that both Δ⁹-THC and CBD have been the subject of numerous scientific investigations aimed to explore their pharmacological properties and biomedical applications.

The pharmacological importance of cannabidiol (CBD) has been in study for several years. CBD is the major non-psychoactive constituent of plant *Cannabis sativa* and its coadministration with Δ⁹-THC is associated with reduced psychotropic side effects. Currently, CBD is undergoing a lot of research which suggests that it has no addictive effects, good safety profile and has exhibited powerful therapeutic potential in several vital areas. It has wide spectrum of action because it acts through endocannabinoid receptors such as CB₁ and it also may act on other receptors, such as GPR55, 5HT1A, and TRPV1 (Morales et al. Front Pharmacol. 2017; 8:422). This indicates its therapeutic value for numerous medical conditions because of its neuroprotective and immunomodulatory properties. Potential therapeutic applications of CBD include, analgesic, anti-inflammatory, anxiolytic, anti-arthritic, antidepressant, anti-Alzheimer disease, anti-ischemic, anti-epilepsy and neuroprotective (Morales et al. Front Pharmacol. 2017; 8:422). More promising areas appear to include diabetes and cancer where CBD exhibits lesser side effects and more therapeutic benefits as compared to recent available medical therapies (Russo et al. Adv Pharmacol. 2017; 80:67-134). Hence, CBD is a promising substance for the development of new drugs. Is has been disclosed that CBD induces cell death of activated hepatic stellate cells that plays a role on liver fibrosis (Lim et al., Cell Death Dis. 2011; 2(6): e170). In addition. CBD attenuates diabetes-associated myocardial fibrosis (Rajesh et al., J Am Coll Cardiol. 2010; 56(25): 2115-2125). However, the benefit of CBD on specific fibrotic diseases such as Scleroderma and many other fibrotic diseases has not been disclosed.

Cannabidiol (CBD) is normally taken to refer to the naturally occurring (-) enantiomer. However, (+)CBD has been synthesized (Hanus et al. Org Biomol Chem. 2005;3(6):1116-23) but has received little attention. Unlike (-)CBD, (+)CBD has been shown to have affinity at CB₁ and CB₂ receptors, whereas both compounds inhibited anandamide hydrolysis and modulated the activity of the TRPV-1 (Bisogno et al. Br J Pharmacol. 2001;134(4):845-52). The (+)CBD isomer was more active than the (-)CBD isomer as an anticonvulsant agent in a mouse seizure model (Leite, J., et al. Pharmacology. 1982;24(3):141-6), suggesting that the (+)CBD enantiomer is also endowed with pharmacological activities that are of interest for the development of novel therapies.

Fibrosis is a wound-healing response to either acute or chronic cellular injury that is characterized by the accumulation of extracellular matrix (ECM). Evidences have demonstrated that fibrogenesis is associated with renin-angiotensin system, inflammation and oxidative stress, transforming growth factor β (TGF-β)/Smad signaling, Wnt/β-catenin signaling and lipid metabolism. Among them, TGF-β/Smad signaling plays an important role in fibrosis (Hu et al., Chem Biol Interact. 2018; 292:76-83). In recent years, more attention has been paid to TGF-β/Smad signaling pathway as an effective target of anti-fibrotic therapy (McVicker et al. Front Pharmacol. 2017; 8:318). TGF-β is a multi-functional mediator expressed in endothelial, hematopoietic, and connective tissue cells. It is well known that TGF-β exerts its biological effects by activating downstream mediators including Smad2 and Smad3 and under pathological conditions both Smad2 and Smad3 expression are upregulated. It is now well accepted that TGF-β is a key mediator in the development of fibrosis and inflammation in diseases such as renal fibrosis, hepatic fibrosis, skin fibrosis, pulmonary fibrosis and others organ fibrosis (McVicker et al. Front Pharmacol. 2017; 8:318). The blockade of TGF-β by neutralizing TGF-β antibodies, decorin, and antisense oligonucleotides prevents or ameliorates fibrosis (Varga et al. Curr Opin Rheumatol. 2008 Nov; 20(6): 720-728; McVicker et al. Front Pharmacol. 2017; 8:318).

Systemic sclerosis (SSc) or scleroderma is an autoimmune multi-organic complex connective tissue disease associated with high mortality (Fuschiotti P. 2016; 5:21-35). SSc patients fall into two clinical forms: limited cutaneous (lcSSc) and diffuse cutaneous systemic sclerosis (dcSSc). The lcSSc form is characterized by the involvement mainly of the skin and the predominance of vascular complications over fibrosis. The damage to internal organs occurs later and is less severe. On the other hand, the dcSSc is the most severe form of the disease and involves extensive skin fibrosis, accompanied by the involvement of internal organs such as the lungs, kidneys and esophagus, among others. Although the pathophysiology of the disease in not completely understood, it is known that SSc is the result of three main pathological processes: (1) innate and adaptive immune response that promotes the production of autoantibodies, (2) microvascular endothelial cell damage and (3) fibroblast dysfunction leading to excessive accumulation of collagen and other elements of the extracellular matrix contributing to fibrosis, that is the hallmark of SSc. SSc is initiated by microvascular injury and inflammation followed by fibroblast activation, a key event in fibrosis development (Pattanaik et al. Front Immunol. 2015; 6:272). Activated fibroblasts are more resistant to apoptosis and differentiate to myofibroblasts, which are responsible for the excessive collagen synthesis and TGFβ production (Jelaska et al. Arthritis and rheumatism, 2000; 43, 2230-2239). Excessive TGFβ signaling is the hallmark of SSc and different strategies aimed to disrupt the TGFβ/SMAD signaling pathway have been proposed for the treatment of SSc and related fibrotic diseases (Varga et al. Rheumatology, 2009;5, 200-206).

The main feature of SSc is the hardening of the skin, and the ECS has been identified throughout different cell populations in both mouse and human. The ECS plays a role in all the critical processes involved in the development of SSc, from angiogenesis to cellular differentiation and apoptosis, metabolism and immune function. Cannabinoids exert immunosuppressive and anti-inflammatory activity on the immune system. Macrophage and mast cell degranulation represents an important source of profibrotic factors during fibrosis (Gruber et al. Curr Rheumatol Rep. 2003;5(2):147-53; Wynn et al. Semin Liver Dis. 2010;30(3):245-57), and activation of mast cells was prevented by CB₁ blockade (Sugawara et al. J Allergy Clin Immunol. 2013;132(1):182-93). The antifibrotic effects of some cannabinoids could be attributed to different signaling pathways, both dependent and independent of cannabinoid receptors. The fibroblast resistance to apoptosis is a characteristic feature in SSc (Jelaska et al. Arthritis Rheum. 2000;43(10):2230-9). In this context, treatment with WIN 55,212-2, a synthetic cannabinoid with agonistic activity for both cannabinoid receptors, increased SSc fibroblast apoptosis without affecting healthy fibroblasts, and this effect was not reverted in the presence of selective cannabinoid receptor antagonists (Garcia-Gonzalez et al. Rheumatology (Oxford). 2009;48(9):1050-6). Both CB₁ and CB₂ receptors are overexpressed in SSc fibroblast, and that they have shown opposite effects on fibrosis development. CB₁ deficient mice were protected against experimental fibrosis by an indirect mechanism through the reduction of leukocyte infiltrates in the skin (Marquart et al. Arthritis Rheum. 2010;62(11):3467-76). The inflammatory infiltrate releases different cytokines, like IL-4 and IL-13, which promote the synthesis of collagen by fibroblasts (O'Reilly et al. Rheumatology. 2012;51(9):1540-9). Blocking CB₁ would be beneficial in the early stages of SSc where inflammation is a predominant phenomenon. Accordingly, the activation of CB₁ exacerbated mouse experimental fibrosis induced by bleomycin (BLM) (Marquart et al. Arthritis Rheum. 2010;62(11):3467-76), and FAAH knockout mice with higher levels of endocannabinoids were more susceptible to BLM-induced fibrosis via CB₁ activation. Moreover, SSc fibroblasts showed decreased levels of FAAH (O'Reilly et al. Rheumatology. 2012;51(9):1540-9).

The role of epidermal cells in SSc and skin fibrosis has been also investigated (McCoy et al. Rheumatology. 2017; 56(11):1970-1981). In this sense, keratinocytes, the main cell type of the epidermis, are implicated in the pathogenesis of SSc, c-Rel, a subunit of nuclear factor κB (NF-κB), has been found to have abnormal expression in SSc keratinocytes and c-Rel null mouse models of bleomycin-induced skin fibrosis are protected (Fullard et al. Am J Pathol. 2013. 182(6):2109-20). Keratinocyte growth factor, which is increased in SSc fibroblasts, stimulates normal keratinocytes to secrete oncostatin M, which activates fibroblasts (Canady et al. J Invest Dermatol. 2013;133(3):647-657). These data highlight the potential for crosstalk between keratinocytes and the dermis.

There is growing evidence that environmental factors have an impact on alterations and modulation of epigenetic determinants, resulting in SSc onset and progression. A marked correlation has thus been found between SSc onset and occupational exposure to crystalline silica and the following organic solvents: white spirit, aromatic solvents, chlorinated solvents, trichloroethylene, and ketones; the risk associated with high cumulative exposure to silica and organic solvents further appears to be strongly increased in SSc. Environmental factors induce reactive oxygen species (ROS) in epidermal keratinocytes that in turn may interact with fibroblasts promoting fibrogenesis. Thus, dysregulations in the oxidant/antioxidant balance are known to be a major factor in the pathogenesis of the disease. For instance, ROS trigger neoepitopes leading to a breach of immune tolerance and autoimmune responses, activate fibroblasts to proliferate and to produce excess of type I collagen. ROS also alter endothelial cells leading to vascular dysfunction (Grygiel-Gorniak et al. Mediators Inflamm 2014:389582; Sambo et al. Arthritis Rheum, 2001; 44(11):2653-64). Numerous studies have reported a defect in GSH in SSc animal models and humans, but the origin of this defect remains unknown. The transcription factor NRF2 is a key player in the antioxidant defense, as it can induce the transcription of antioxidant and cytoprotective genes, including GSH, through its interaction with the antioxidant response elements. It has been reported that the Nrf2 pathway is highly dysregulated in human and SSc mice with deleterious consequences on fibrosis and inflammation and that Nrf2 modulation represents a therapeutic target in SSc and fibrotic diseases (Kavian et al. Front Immunol. 2018; 9:1896; Wei et al. Transl Res. 2017; 183:71-86. e1).

Pain and itch are common symptoms reported by patients with systemic sclerosis (SSc; scleroderma), which can markedly diminish function and health-related quality of life (HRQL). Pruritus, or itchy skin, is caused by irritation in the skin from the underlying inflammatory process associated with scleroderma. Inflammation is accompanied by the degranulation of mast cells inducing the release of histamine that is a major mediator or itching. Histamine is known to excite a subset of sensory neurons, predominantly C-fibers and it has been shown that histamine activates transient receptor potential vanilloid receptor-1 (TRPV1) in sensory neurons via phospholipase A2 (PLA2) (Shim et al. J Neurosci. 2007;27(9):2331-7). In addition, TRPV-1 is involved in the transmission of pain and the pharmaceutical development of TRPV-1 antagonists as a new class of analgesic agents has been pursued aggressively across the pharmaceutical industry (Moran et al. Br J Pharmacol. 2018; 175(12):2185-2203).

### Summary of Invention

The technical problem to be solved can be regarded as providing compounds and pharmaceutically compositions to be topically administered to an animal in the treatment and/or prevention of fibrotic diseases and/or in the treatment and/or prevention of fibrotic symptoms of fibrotic diseases.

The problem is solved by the compounds and pharmaceutical compositions as defined in the claims for use in the treatment and/or prevention of fibrotic diseases and/or in the treatment and/or prevention of fibrotic symptoms of fibrotic diseases in an animal, wherein said compound or pharmaceutical composition is topically administered to the animal.

Said compound is selected from the group consisting of a compound of Formula Ia or Ib 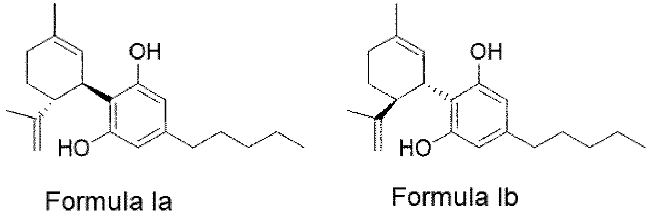 and any pharmaceutically acceptable salt or solvate thereof.

Compound of Formula Ia is (-)Cannabidiol, also referred herein as (-)CBD and compound of Formula Ib is (+)Cannabidiol, also referred herein as (+)CBD.

Pharmaceutical compositions according to present invention comprise said compound Ia or Ib and at least a pharmaceutically acceptable excipient or carrier.

Said compound Ia or Ib and said pharmaceutical compositions comprising the same show capacity to antagonize CB₁ and TRPV-1 receptors, activate Nrf2 pathway, inhibit myofibroblast differentiation, inhibit TGFβ-induced collagen gene transcription and secretion, and show antifibrotic and anti-inflammatory activities *in vivo.*

Said compound Ia or Ib and said pharmaceutical composition comprising the same are, as demonstrated by the examples of the invention, useful for the treatment and/or prevention of fibrotic diseases and/or in the treatment and/or prevention of fibrotic symptoms of fibrotic diseases.

Also, the technical problem to be solved can be regarded as providing cosmetic compositions and cosmetic uses by topical administration to a subject for preventing keloids, increasing skin elasticity and/or reducing skin itching and/or reducing skin pain.

The problem is solved by the cosmetic composition as defined in the claims and cosmetic uses thereof for preventing keloids, increasing skin elasticity and/or reducing skin itching and/or reducing skin pain in a subject, wherein said compound or cosmetic composition is topically administered to said subject.

The compounds and cosmetic compositions of the present invention increase red blood cell deformability and reduce itching and pain by inhibiting histamine release by skin mast cells and antagonizing peripheral sensory TRPV-1 receptor.

### Detailed description of Invention

The invention provides a compound or a pharmaceutical composition comprising said compound and at least a pharmaceutically acceptable excipient or carrier, for use in the treatment and/or prevention of fibrotic diseases and/or in the treatment and/or prevention of fibrotic symptoms of fibrotic diseases in an animal, wherein said compound or pharmaceutical composition is topically administered to the animal, wherein said compound is selected from the group consisting of a compound of Formula Ia or Ib 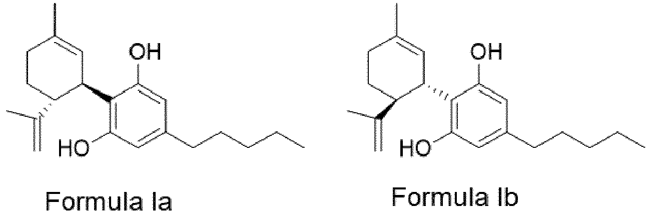 and any pharmaceutically acceptable salt or solvate thereof and wherein said fibrotic diseases and/or fibrotic symptoms of fibrotic diseases are selected from the group consisting of scleroderma, eosinophilic fasciitis, lupus, discoid lesions associated with lupus, discoid lesions associated with surgical adhesions, mixed connective tissue disease, fibrodysplasia, fibrocystic disease, sarcoidosis, myositis, inclusion body myositis, polymyositis, primary idiopathic polymyositis, childhood polymyositis, dermatomyositis, childhood dermatomyositis, primary idiopathic dermatomyositis in adults, carpal tunnel syndrome, Dupuytren's contracture, Limited Joint Mobility, vasculitis, coronary artery vasculitis, polyarteritis nodosa, temporal arteritis, cerebrosclerosis, annular sclerosis, diffuse sclerosis and lobar sclerosis.

In an embodiment, compounds Ia or Ib or compositions comprising the same are for use in amelioration of fibrotic diseases and/or in the amelioration of fibrotic symptoms of fibrotic diseases in an animal.

As used herein, the term "amelioration" refers to reducing the rate of progression of a disease.

In an embodiment, the invention refers to a method of treating and/or preventing fibrotic diseases and/or to a method of treating and/or preventing fibrotic symptoms of fibrotic diseases in an animal, comprising topically administering an effective amount of said compound Ia or Ib or a composition comprising the same to the animal.

In an embodiment, the invention refers to the use of said compound Ia or Ib or a composition comprising the same for the manufacture of a medicament for the treatment and/or prevention of fibrotic diseases and/or in the treatment and/or prevention of fibrotic symptoms of fibrotic diseases in an animal.

In an embodiment, fibrotic diseases refer to diseases associated with the formation of collagen in excess. Said diseases associated with the formation of collagen in excess refer to scleroderma, mixed connective tissue disease, fibrodysplasia, fibrocystic disease, sarcoidosis, myositis (e.g. polymyositis, primary idiopathic polymyositis, childhood polymyositis, dermatomyositis, childhood dermatomyositis, primary idiopathic dermatomyositis in adults, inclusion body myositis, polymyositis or dermatomyositis associated with malignant tumors). Dermatomyositis can be associated with fibrosing or hypertrophic aspects. In an embodiment, fibrotic symptom refers to fibrotic hypertrophy associated to diseases associated with the formation of collagen in excess.

In an embodiment, fibrotic diseases refer to diseases that have as symptom fibrotic vascular intimal hypertrophy. Said diseases that have as symptom fibrotic vascular intimal hypertrophy refer to vasculitis (including coronary artery vasculitis), polyarteritis nodosa or temporal arteritis. In an embodiment, fibrotic symptoms refer to vascular intimal hypertrophy symptoms.

In an embodiment, fibrotic diseases refer to diseases that have as symptom fibrotic hypertrophy of skin and/or of muscle tissue. Said diseases that have as symptom fibrotic hypertrophy of skin and/or of muscle tissue refer to scleroderma, eosinophilic fasciitis, discoid lesions associated with lupus or discoid lupus or surgical adhesions. In an embodiment, fibrotic symptoms refer to hypertrophy, fibrotic hypertrophy, or fibrosis of skin or muscle tissue.

In an embodiment, fibrotic diseases include diseases that have as symptom fibrotic hypertrophy of nerve tissue. Said diseases that have as symptom fibrotic hypertrophy of nerve tissue refer to cerebrosclerosis, annular sclerosis, diffuse sclerosis and lobar sclerosis. In an embodiment, fibrotic symptom is hypertrophy, fibrotic hypertrophy, or fibrosis, of nerve tissue. In an embodiment, fibrotic symptoms refer to fibrotic and cytokine-induced elements of carpal tunnel syndrome.

In an embodiment, fibrotic diseases refer to Dupuytren's contracture, a contracture of the palmar fascia often causing the ring and little fingers to bend into the palm. In an embodiment, fibrotic symptoms refer to hypertrophy, fibrotic hypertrophy or fibrosis of Dupuytren's contracture,

Limited Joint Mobility (LJM) is a disorder associated with diabetes and typically involves the joints of the hands. The fourth and fifth fingers are affected initially by limitation of motion. AGE glycation and crosslinking of tendons (collagen) in the joints is believed to contribute to the disease. In an embodiment, fibrotic symptoms refer to inelasticity, fibrous tissue or cytokine-induced inflammation associated with limited joint mobility.

In an embodiment, fibrotic symptom refers to fibrotic hypertrophy associated to autoimmune diseases affecting the skin.

In an embodiment, said animal is a human.

In an embodiment, pharmaceutical compositions comprising compound Ia or Ib may consist of sterile solutions in water, saline solutions or solutions buffered to physiological pH.

In an embodiment, pharmaceutical compositions comprising compounds Ia or Ib may include various thickeners, buffers, preservatives, surfactants, nanoparticles, liposomes and others. Said pharmaceutical composition may further include active ingredients such as antimicrobial agents, anti-inflammatory agents, anaesthetic agents, etc.

In an embodiment, pharmaceutical compositions comprising compounds Ia or Ib can be in crystalline, powder, granular, compacted solid, liquid, solution, suspension, elixir, syrup, emulsion, cream, gel, droplet, mist, vapor or spray form. Conventional techniques for the preparation of pharmaceutical compositions may be used. For example, said pharmaceutical composition may be comprised in an ampoule, sachet, syringe, cartridge, nebulizer or other container.

In an embodiment, compounds Ia or Ib may be slowly released or sustained released in a pharmaceutical composition.

In an embodiment, compounds Ia or Ib may be released so that a constant dose is achieved in a pharmaceutical composition.

In an embodiment, pharmaceutical compositions comprising compound Ia or Ib may contain sterile aqueous or non-aqueous solutions, suspensions and emulsions, and it may also contain buffers, diluent additives and others. Examples of non-aqueous solvents are: propylene glycol, polyethylene glycol and vegetable oils such as olive oil. Examples of aqueous solvents are: water, alcohol-aqueous solutions, emulsions or suspensions, including saline and buffer solutions. Preservatives and other additives may also be present, such as, for example, antimicrobial agents, antioxidants, chelating agents, inert gases, etc.

In an embodiment, pharmaceutical compositions comprising compound Ia or Ib can be in the form of creams, lotions, gels, drops, suppositories, sprays, liquids and powders. Conventional pharmaceutical carriers, aqueous bases, oily bases and thickeners may also be present. Thickening, flavouring, diluent, emulsifying, and dispersant agents may also be present.

### Combination Therapies

In vascular-related fibrotic therapies, compound Ia or Ib or, or the compositions comprising the same can be administered concurrently or in a combined formulation with one or more antioxidants. Examples of appropriate antioxidants are vitamin A, vitamin B6, vitamin C, vitamin E, glutathione, β-carotene, α-lipoic acid, coenzyme Q10, selenium and zinc, which are administered in effective amounts as it is well known in the art. Thus, in an embodiment, pharmaceutical compositions of the invention comprise an effective amount of an antioxidant.

In an embodiment, compound Ia or Ib combined with at least an antioxidant is for the treatment of vascular-related fibrotic therapies.

In an embodiment, pharmaceutical compositions comprising compound Ia or Ib and at least an antioxidant are for the treatment of vascular-related fibrotic therapies.

In an embodiment, said antioxidant is selected from the group consisting of vitamin A, vitamin B6, vitamin C, vitamin E, glutathione, β-carotene, α-lipoic acid, coenzyme Q10, selenium and zinc.

For treating scleroderma, the invention further provides pharmaceutical compositions comprising a compound Ia or Ib of the invention in combination with an effective amount of a PPARγ agonist to be used topically.

In an embodiment, compound Ia or Ib combined with a PPARγ agonist is for the treatment of scleroderma.

In an embodiment, pharmaceutical compositions comprising compound Ia or Ib and a PPARγ agonist are for the treatment of scleroderma.

For treating skin fibrotic conditions associated to autoimmune diseases, the invention further provides pharmaceutical compositions comprising compounds Ia or Ib in combination with immunosuppressive drugs and/or Vitamin D3, and/or histone deacetylase inhibitors (HDACi).

In an embodiment, said compound Ia or Ib combined with at least an immunosuppressive drug, Vitamin D3, or a histone deacetylase inhibitor (HDACi), or combinations thereof is for treatment of fibrotic hypertrophy associated to autoimmune diseases affecting the skin.

In an embodiment, said pharmaceutical compositions comprising compound Ia or Ib and at least an immunosuppressive drug, Vitamin D3 or a histone deacetylase inhibitor (HDACi) or combinations thereof are for the treatment of fibrotic hypertrophy associated to skin autoimmune diseases.

In an embodiment, the treatment with compound Ia or Ib or pharmaceutical composition comprising the same is combined with a treatment with a PPARγ agonist, an immunosuppressive drug, vitamin D3, a histone deacetylase inhibitor (HDACi), or combinations thereof.

### Cosmetic uses

Compound Ia or Ib, or cosmetic compositions comprising the same, topically administered to the skin of a subject, are suitable for cosmetic purposes, such as prevention of keloids and increasing skin elasticity and/or reducing skin itching and/or reducing skin pain in said subject.

This cosmetic use refers to a non-therapeutic use and should be understood as excluding therapeutic uses of compound Ia or Ib.

The invention also refers to a cosmetic composition comprising a compound selected from the group consisting of a compound of Formula Ia or Ib and any cosmetically acceptable salt or solvate thereof, and at least a cosmetically acceptable excipient or carrier, wherein said cosmetic composition is topically administered to the skin of a subject for preventing keloids, increasing skin elasticity and/or reducing skin itching and/or reducing skin pain in said subject.

In an embodiment, the invention relates to the use of compound Ia or Ib or a cosmetic composition comprising the same and at least a cosmetically acceptable excipient or carrier, wherein compound Ia or Ib or cosmetic composition are topically administered to the skin of a subject for preventing keloids, increasing skin elasticity and/or reducing skin itching and/or reducing skin pain in said subject.

### Definitions

As used herein, the terms "fibrotic diseases" and "fibrotic symptoms of fibrotic diseases" refer to scleroderma, eosinophilic fasciitis, lupus, discoid lesions associated with lupus, discoid lesions associated with surgical adhesions, mixed connective tissue disease, fibrodysplasia, fibrocystic disease, sarcoidosis, myositis, inclusion body myositis, polymyositis, primary idiopathic polymyositis, childhood polymyositis, dermatomyositis, childhood dermatomyositis, primary idiopathic dermatomyositis in adults, carpal tunnel syndrome, Dupuytren's contracture, Limited Joint Mobility, vasculitis, coronary artery vasculitis, polyarteritis nodosa, temporal arteritis, cerebrosclerosis, annular sclerosis, diffuse sclerosis and lobar sclerosis.

As used herein, the term "pharmaceutically acceptable excipient" refers to a compound included in a pharmaceutical composition for the purpose of long-term stabilization or to confer a therapeutic enhancement on the active ingredient in the final dosage form, such as facilitating drug absorption, reducing viscosity, or enhancing solubility. Excipients can also be useful in the manufacturing process, to aid in the handling of the active substance concerned. Excipients can also be useful for preventing denaturation or aggregation of the components of the pharmaceutical composition. The excipient may be an inert ingredient or ingredients. Said excipient or carrier may be a diluent, as a way of example.

As used herein, the term "pharmaceutically acceptable carrier" refers to a substance which promotes the delivery of active agents into cells or promotes efficient crossing of biological membranes.

As used herein, the term "antioxidant" refers to a compound that prevents oxidation. An antioxidant acts as preservative.

As used herein, the term "PPARγ agonist" refers to drugs which act upon the peroxisome proliferator-activated receptor. They are used for the treatment of symptoms of the metabolic syndrome, mainly for lowering triglycerides and blood sugar.

As used herein, the term "immunosuppressive drug" refers to drugs that inhibit or prevent activity of the immune system. They are used in immunosuppressive therapy to prevent the rejection of transplanted organs and tissues (e.g., bone marrow, heart, kidney, liver) and treat autoimmune diseases or diseases that are most likely of autoimmune origin, (e.g., scleroderma, lupus and sarcoidosis).

As used herein, the term "histone deacetylase inhibitor (HDAC inhibitors, HDACi)" refers to compounds that inhibit histone deacetylases. HDACi have been used in psychiatry and neurology as mood stabilizers, anti-epileptics, treatments for cancers, parasitic and inflammatory diseases.

As used herein, the term "medical use" refers to compound Ia or Ib for use in the treatment or prevention of fibrotic diseases or fibrotic hypertrophy in an animal by topical administration of compound Ia or Ib.

As used herein, the term "cosmetic use" refers to the use of compound Ia or Ib for increasing skin elasticity and/or reducing skin wrinkles and/or reducing skin itching and/or reducing skin pain by topical administration of compound Ia or Ib to the skin of a subject. Said cosmetic use refers to a non-therapeutic use and should be understood as excluding therapeutic uses of compound Ia or Ib.

### Brief description of the figures

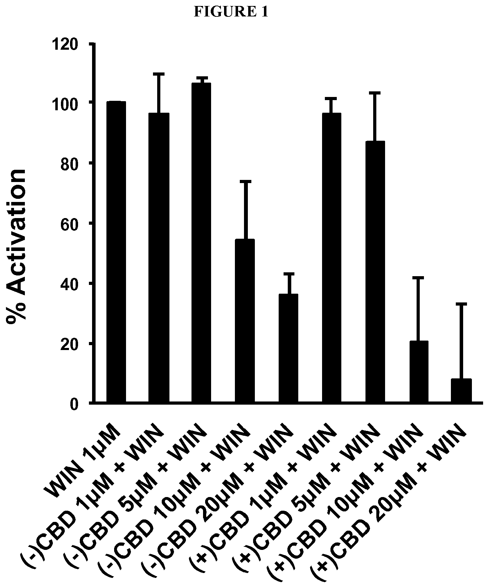

**Figure 1****. CB₁ antagonism assay in HEK293T-CB₁ cells**
   (-)CBD and (+)CBD inhibits WIN55,212-induced activation of CRE-luc in HEK293T-CB₁ cells transfected with the CRE-Luc plasmid. The concentration of the tested compounds (µM) is shown at the X-axis and the percentage of CRE-Luc activation is shown at the Y-axis, considering the induction mediated by WIN55,212 (1 µM) as 100% activation over untreated cells.
**Figure 2****. TRPV-1 antagonism assay in HEK-293T-TRPV1 cells**
   **(A)** (-)CBD inhibits intracellular calcium mobilization induced by capsaicin in HEK-293T-TRPV cells. **(B)** (+)CBD and (-)CBD inhibit capsaicin-induced cell death in HEK-293T-TRPV1 cells.
**Figure 3****. Col1A2 gene transcriptional activity in NIH-3T3-Col1A2 cells**
   NIH-3T3-Col1a2-luc cells were pre-treated with (-)CBD and (+)CBD for 1 h and stimulated with TGFβ1 for the following 24 h (n=5). Results are represented as mean percentage of inhibition ± S.D. taking TGFβ as 100%. *p<0.05 versus TGFβ1.
**Figure 4****. Collagen deposition assay in normal human dermal fibroblasts (NHDFs)**
   NHDFs were serum starved (1% FBS) for 24 h. Then, cells were pre-treated with (-)CBD (A) and (+)CBD (B) for 1 h and stimulated with TGFβ for 24 h. Soluble collagen in the culture medium was measured using the Sircol assay. Results are represented as mean percentage of inhibition ± S.D. taking TGFβ as value 1. * p<0.05 versus TGFβ1.

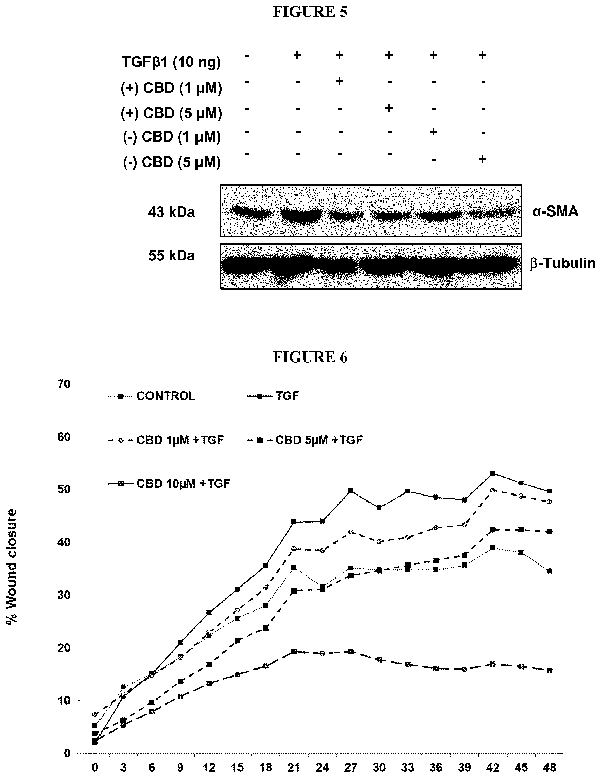

**Figure 5****. Determination of α-SMA expression in NIH-3T3 cells**
   Stimulation of NIH-3T3 cells with TGFβ in the absence and the presence of (-)CBD or (+)CBD during 24 h, and the expression of α-SMA and α-tubulin determined by Western blots.
**Figure 6****. Fibroblast migration assay in fibroblasts**
   Scratch assay on NHDFs cells treated with either TGFβ in the absence or the presence of increasing concentrations of (-)CBD. Results were plotted using the Incucyte^{FLR} software in terms of percentage of relative wound density ±S.D. as a function of time (n = 3). *p < 0.05 **p < 0.01 versus control; ^{##}p < 0.01 versus TGFβ or IL-4 treated cells.

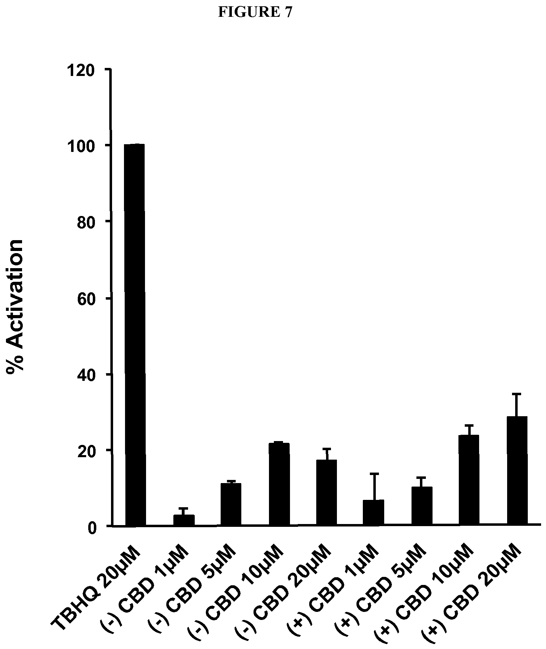

**Figure 7****. Nrf2 activation in keratinocytes**
   Luciferase activity assay on HaCaT-ARE-Luc cells treated with increasing concentrations of the CBD isomers for 6 h and using as a positive control cells treated with 0,01 mM of Tert-butyl-hydroperoxide (TBHP) for 6 h. Specific transactivation is expressed as fold induction over basal levels (untreated cells). Both (+)CBD and (-)CBD were able to activate the Nrf2 pathway in keratinocytes and in a concentration-dependent manner.

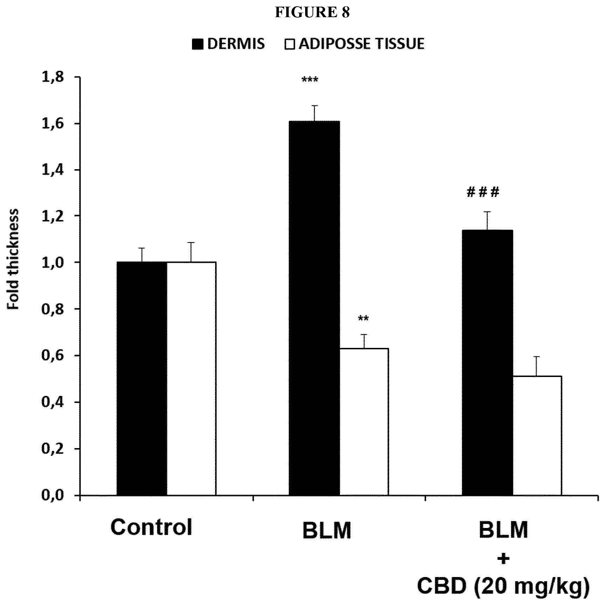

**Figure 8****. Antifibrotic activity of (-)CBD in vivo**
   Mice were injected with bleomycin (BLM) for three weeks and treated in parallel with i.p. injections of (-)CBD (20 mg/Kg). Skin sections were stained with Masson's trichrome and their respective measurement of dermal and subcutaneous adipose layer thickness is shown. Results are represented as mean ± SEM referred to control group (n=9 animals per group). * p<0.05 versus control; ^{#}p<0.05 versus BLM-treated mice.

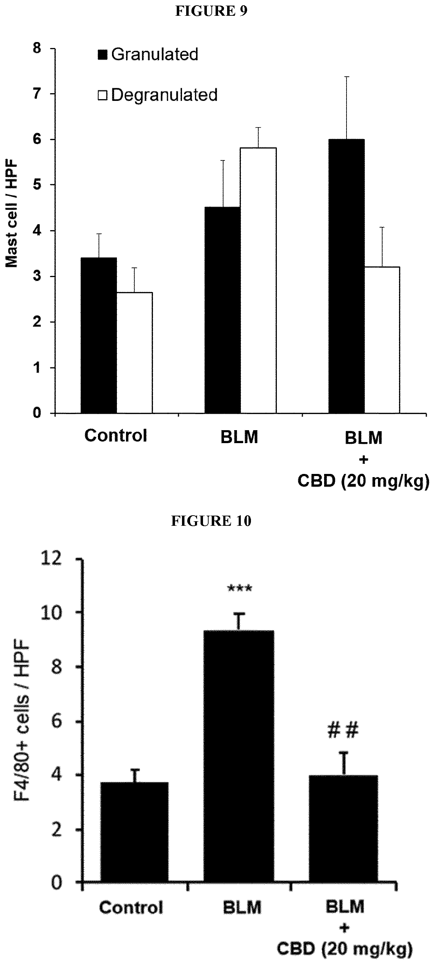

**Figure 9****. Effect of (-)CBD on mast cell degranulation in vivo**
   Mice were injected with BLM for three weeks and treated in parallel with i.p. injections of (-)CBD (20 mg/Kg). Skin sections were stained with toluidine blue and mast cell degranulation (granulated vs degranulated) quantified and expressed as the number of Mast cells/HPF. Results represent the mean ± SEM. ** p<0.01 versus control; ^{#}p<0.05 versus BLM-treated mice.
**Figure 10****. Effect of (-)CBD on skin macrophage infiltration in vivo**
   Mice were injected with BLM for three weeks and treated in parallel with i.p. injections of (-)CBD (20 mg/Kg). F4/80⁺ macrophages in the skin were detected by immunostaining, quantified and expressed as F4/80⁺cells/HPF. *** p<0.001 versus control; ^{##}p<0.01 versus BLM-treated mice.

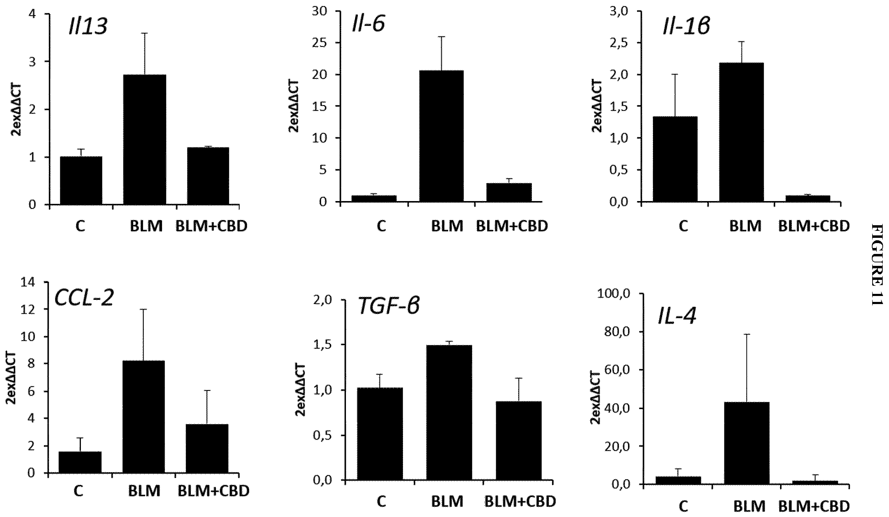

**Figure 11****. (-)CBD reduces the expression on inflammatory marker mRNAs in the skin**
   Gene expression of inflammatory markers including 1113, IL1β, IL6, CCL2, TGFβ and IL4, was down regulated by (-)CBD in bleomycin-challenged mice compared to control BLM. Expression levels were calculated using the 2^{-ΔΔCt} method. Values are expressed as means ± SEM for 3 animals *per* group.

### Detailed description of embodiments of the Invention

### Examples

The examples of the present invention described below aim to illustrate its preferred embodiments without limiting its scope of protection.

### Example 1. Enantiomers (+)CBD and (-)CBD are antagonists of CB₁ and TRPV-1 receptors

To investigate the biological activities of the CBD enantiomers on CB₁ and TRPV1 receptors were performed functional assays in both HEK-293T-CB₁ and HEK-293T-TRPV1 cells, which overexpress human CB₁ and TRPV-1 receptors respectively. HEK-293T-CB₁ (stably transfected with CB₁ cDNA) were maintained in supplemented DMEM medium containing 10% FBS and 1% antibiotics penicillin/streptomycin (DMEM complete medium) at 37°C in a humidified atmosphere of 5% CO₂. The cells were incubated in 24-well plates (1x10⁵ cell/ml) and transiently transfected with 0,1 µg/ml of the plasmid CRE-Luc that contains six consensus cAMP responsive elements (CRE) linked to firefly luciferase. Transient transfections were performed with Rotifect (Carl Roth GmbH, Karlsruhe, Germany) according to the manufacturer's instructions and harvested 24 h after transfection. For CB₁ antagonistic activity the transfected cells were incubated with increasing concentrations of the CBD enantiomers for 15 min and then stimulated with WIN-55, 212-2 (CB₁ agonist), during 6 h and then luciferase activity measured in the cell lysates. WIN-55, 212-2 ((*R*)-(+)-[2,3-Dihydro-5-methyl-3[(4-morpholinyl)methyl]pyrrolo[1,2,3-de]-1,4-benzoxazinyl]-(1-naphthalenyl)methanone mesylate salt) and (-)CBD (IUPAC name: 2-[(1R,6R)-3-methyl-6-prop-1-en-2-ylcyclohex-2-en-1-yl]-5-pentylbenzene-1,3-diol) were purchased from Cayman Chemicals (Ann Arbor, MI, USA) and (+)CBD was obtained from Symrise Co (Holzminden, Germany). Both (+)CBD and (-)CBD showed a clear CB₁ antagonistic activity (Figure 1).

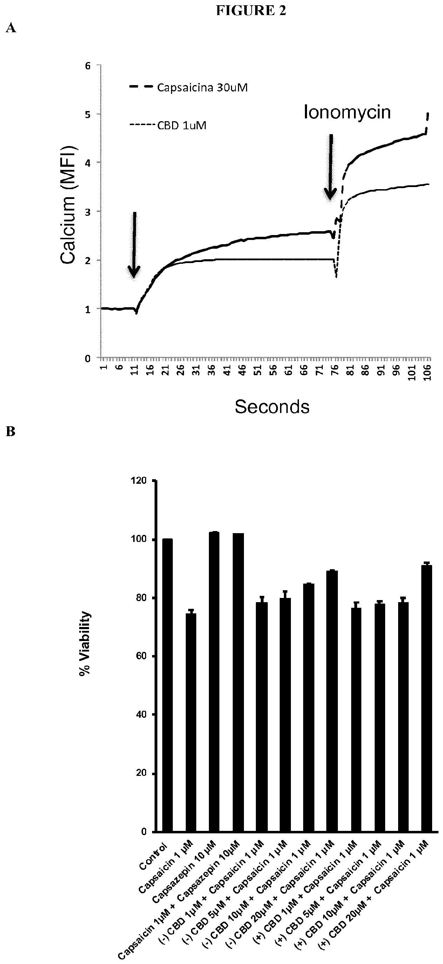

Next, the effect of (+)CBD and (-)CBD on TRPV-1 activity was investigated. TRPV-1 is an ionotropic receptor that upon activation by a specific agonist such as capsaicin mobilize intracellular calcium in TRPV-1 expressing cells. Moreover, TRPV-1-induced calcium exerts cytotoxicity in non-neuronal cells overexpressing this receptor. To explore the effect of (+)CBD on calcium mobilization HEK-293T-TRPV1 cells were cultured in 96-well plates (10³ cells/well) and loaded with Fura 2 acetyoxymethyl ester (Fura-2 AM; 8 µM) (Molecular Probes, Carlsbad, CA) in Dulbecco's modified Eagle's medium (containing 110 mg/l pyruvate) supplemented with 10% FBS and incubated (40 min, 37°C, 5% CO₂). The cells were preincubated with (+)CBD and after 15 sec treated with capsaicin (1µM). Changes in intracellular free calcium concentration (intracellular [Ca²⁺]_{free}) were measured by digital microscopy equipped with ratiometric recording of single cells. (BD Pathway 855 Bioimager). The field of cells was monitored by sequential dual excitation, 352 and 380 nm, and the analysis of the image ratios used methods described previously to calculate changes in intracellular [Ca²⁺]_{free}. The ratio images were acquired every 6 s. At the end of experiments, the cells were exposed to ionomycin (30 s, 1 µM) to obtain a maximal response. (-)CBD was found to prevent capsaicin-induced calcium mobilization in HEK-293T-TRPV1 cells (Figure 2A).

Capsaicin-induced cytotoxicity as a model of TRPV-1 agonistic assay was also investigated in HEK-293T-TRPV1 cells. The cells (2x10⁴/well) were cultured in triplicate in 96-well microtiter plates in 200 µL of complete medium and incubated with (-)CBD or (+)CBD and then stimulated with the agonist Capsaicin (1 µM) for 3 h. Capzasepine (10 µM) was used as the positive control of TRPV-1 antagonism. Then, TRPV-1 induced cytotoxicity was measured by fluorescence using the die YOYO-1 (Life Technologies). YOYO-1 was diluted in cell culture medium and added to a final concentration of 0.1 µM to both experimental and control wells. YOYO-1 is a cell impermeant cyanine dimer nucleic acid stain that can only enter cells with a compromised plasma membrane and fluorescently stain the nuclear DNA. By adding YOYO-1 to damaged cells an increase in YOYO-1 fluorescence is detected. Treated cells are placed in an Incucyte FLR imaging system, and the YOYO-1 fluorescence is measured after 3 h. Following the incubation period, end point analysis is performed to measure the total number of DNA containing objects by treating cells with 0.0625% triton X-100 to permeabilize the cell membrane. Object counting analysis is performed using the Incucyte FLR software to calculate the total number of YOYO-1 fluorescence positive cells and total DNA containing objects (end point). The cytotoxicity index is calculated by dividing the number of YOYO-1 fluorescence positive objects by the total number of DNA containing objects for each treatment group. Both (-)CBD and (+)CBD were found to prevent capsaicin-induced calcium mobilization in HEK-293T-TRPV1 cells (Figure 2B).

### Example 2. Effect of CBD on collagen gene transcription and secretion in fibroblasts

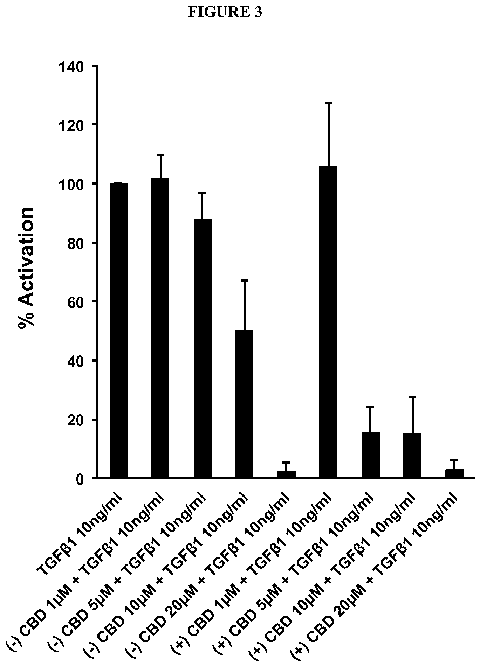

The effect of CBD isomers on collagen transcriptional regulation in NIH-3T3 cells stably expressing the Col1A2-luc plasmid, which encodes the gene promoter of the pro-alpha2 chain component of type I collagen fused to the luciferase gene, was studied. NIH-3T3-COL1A2-Luc fibroblast cells were generated by stable transfection of the COL-1A2-Luc plasmid, containing sequences from -353 to +58 bp of the human COL1A2 promoter fused to the luciferase reporter gene, and pPURO plasmid. Cells are cultivated at a density of 1 x 10⁴ cells/ml for 24 h at 37°C. Then the cells were treated with the CBD isomers at several concentrations and TGFβ (10 ng/ml) for 24 h. Then, the cells are washed with PBS and lysed in 50 µl lysis buffer containing 25 mM Tris-phosphate (pH 7.8), 8 mM MgCl₂, 1 mM DTT, 1% Triton X-100, and 7% glycerol during 15 min. Luciferase activity and protein concentration is measured in the cell lysates, to calculate the RLU/µg of protein and the results expressed as the percentage of induction considering the value obtained with TGFβ as 100% activation. The protein concentration in the cell extracts was measured by the Bradford method (BioRad). Pre-incubation of NIH-3T3-Col1A2 cells with both (-)CBD and (+)CBD significantly reduced collagen promoter transcriptional activity (Figure 3).

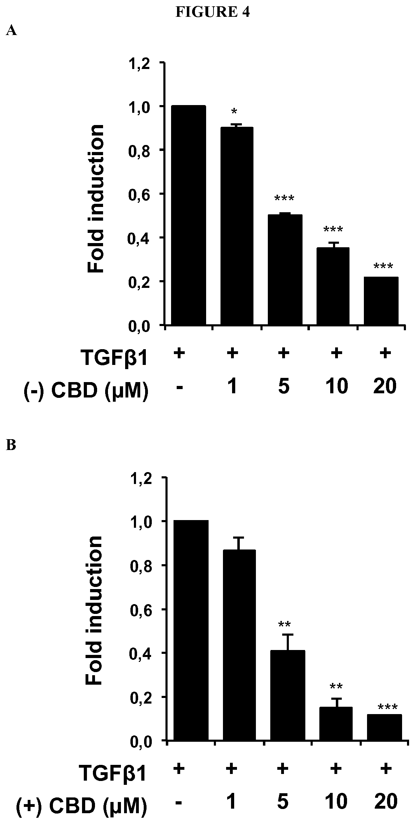

Next, NHDFs were used to correlate the effect of both isomers of CBD on Col1A2 transcriptional activity with collagen secretion. NHDFs were seeded in 24 well plates (5x10⁴ cells/well), pre-treated with either (-)CBD or (+)CBD for 1 h and stimulated with TGFβ for the following 24 h. Collagen secretion was studied by collecting culture medium and measuring soluble collagen using the Sircol Soluble Collagen Assay (Biocolor, County Antrim, UK) following manufacturer's instructions. Both isomers of CBD significantly inhibited TGFβ-induced collagen release and deposition in NHDFs (Figure 4).

### Example 3. Effects of CBD on myofibroblast differentiation and fibroblast migration

It is well known that TGFβ drives the conversion of fibroblasts into myofibroblasts, and the accumulation of myofibroblasts cells in fibrotic skin is a hallmark of SSc. Myofibroblasts are characterized by the novo synthesis of α-smooth muscle actin (a-SMA) fibers. To investigate α-SMA expression NIH-3T3 cells were preincubated with either (+)CBD or (-)CBD for 1 h and then treated with TFGβ (10 ng/ml) for 24 h. Cells were washed with PBS and proteins extracted in 50 µl of lysis buffer (50 mM Tris-HCl pH 7.5, 150 mM NaCl, 10% glycerol and 1% NP-40) supplemented with 10mM NaF, 1 mM Na₃VO₄, 10 µg/ml leupeptine, 1 µg/ml pepstatin and aprotinin, and 1 µl/ml PMSF saturated. Protein concentration was determined by the Bradford assay (Bio-Rad, CA, USA) and 30 µg of proteins were boiled at 95 °C in Laemmli buffer and electrophoresed in 10% SDS/PAGE gels. Separated proteins were transferred to PVDF membranes (20V for 30 min) and blocked in TBS solution containing 0.1% Tween 20 and 5% non-fat dry milk for 1 hour at room temperature. Immunodetection of specific proteins was carried out by incubation with primary antibody against α-SMA (1:500; sc-32251, Santa Cruz). After washing membranes, horseradish peroxidase-conjugated secondary antibody was added and detected by chemiluminescence system (GE Healthcare Europe GmbH). The blot was striped and incubated with an antibody against α-tubulin from Sigma (1:10.000; DM-1A). Densitometric analysis of immunoblots was performed using Image J software (NIH; Bethesda, MD, USA). It was found that both CBD isomers attenuated the expression α-SMA protein induced by TGFβ (Figure 5). These results indicated that CBD prevents TGFβ-induced myofibroblast differentiation.

Migration of skin fibroblast plays a crucial role not only in normal wound healing but also in fibrotic diseases. To further analyze this point, it was tested if the anti-fibrotic effect of (-)CBD could be associated with reduced fibroblast migration. To investigate this possibility, the effect of (-)CBD on TGFβ-induced wound healing was investigated. NHDFs (2x10³ cells) were seeded in complete DMEM in a 96-well Essen ImageLock plates (Essen BioScience). When cells reached confluence, wound scratches were made using the 96-pin WoundMaker (Essen BioScience). Then, culture medium was replaced by DMEM supplemented with 1% antibiotics and with mitomycin C (10 ng/ml) to block cell proliferation. (-)CBD and TGFβ1 10 ng/ml were added in parallel. Wound healing activity was analyzed using IncuCyte HD system by taking images every 3 h for 48 h and the percentage of wound confluence calculated with the IncuCyte software. (-)CBD significantly attenuated wound closure induced by either TGFβ1 or rhIL-4 in a concentration-dependent manner (Figure 6).

### Example 4. CBD isomers activated the Nrf2 pathway

The involvement of inflammation and ROS in scleroderma pathogenesis suggests a potential therapeutic role for compounds that activate Nuclear factor-E2 p45-related factor 2 (Nrf2). Nrf2 is a transcription factor which, upon activation in response to inflammatory or oxidative stress, activates a robust anti-inflammatory and cytoprotective transcription program. Therefore, Nrf2 activation may represent a novel approach for the treatment of scleroderma and other fibrotic diseases. Thus, the effect of CBD isomers on the activation of the Nrf2 pathway in the keratinocyte cell line HaCaT-ARE-Luc, containing the Nqo1 Antioxidant Response Element (ARE)-Luc reporter plasmid stably transfected, was explored. ARE is activated by all members of the CNC family of factors (Nrf1, Nrf2, Nrf3 and p45 NF-E2). The cells are cultivated in 96-well plates at the concentration of 2x10⁴ cells/well in a CO₂ incubator at 37°C. For induction of Nrf2 activation the cells were treated with increasing concentrations of the CBD isomers for 6 h. As a positive control the cells were treated with 0,01 mM of Tert-butyl-hydroperoxide (TBHP) for 6 h. Then the cells were washed twice in PBS and lysed in 25 mM Tris-phosphate pH 7.8, 8 mM MgCl₂, 1 mM DTT, 1% Triton X-100 and 7% glycerol during 15 min at RT in a horizontal shaker. Luciferase activity was measured using TriStar² Berthold/LB942 multimode reader (Berthold Technologies) following the instructions of the luciferase assay kit (Promega, Madison, WI, USA). The background obtained with the lysis buffer was subtracted in each experimental value, and the specific transactivation expressed as fold induction over basal levels (untreated cells). As depicted in Figure 7, both (+)CBD and (-)CBD were able to activate the Nrf2 pathway in keratinocytes and in a concentration-dependent manner.

### Example 5. Treatment based on administration of (-)CBD in skin fibrosis induced in vivo (Bleomycin model)

The bleomycin (BLM) mice model is widely used to evaluate potential anti-inflammatory and anti-fibrotic therapies for SSc. BLM causes inflammation in a short period of time, characterized by elevation of proinflammatory cytokines and growth factors that peak around day 14 of BLM exposure. In the inflammatory model, BLM administration for 3 weeks resulted in increased dermal thickness versus control group, which can be attenuated by anti-fibrotic therapies. The inflammatory model of SSc was used to study (-)CBD preventive effects. Fibrosis was induced by daily subcutaneous administration of BLM (50 µg/mice; 100 µl) (Mylan, Barcelona, Spain) for 3 weeks. Treatments were administered in parallel and consisted of daily intraperitoneal (i.p.) injections of (-)CBD (20 mg/kg), or vehicle (4% DMSO, 6.2% Tween20, saline; 100 µl). Subcutaneous injections of 0.9% NaCl served as a control. Mice were acclimated to manipulators for a week previously to the experiments in order to reduce stress and allow the detection of subtle changes in behavior by researchers. Mice weight was evaluated weakly and no significant changes were observed between experimental groups. No behavioral changes were observed during the protocols. Neither piloerection nor hunched appearances were appreciated during the experimental procedures. Mice were sacrificed by cervical dislocation and the back skin was removed and processed for histological examination or was frozen for further analysis. BLM administration for 3 weeks resulted in increased dermal thickness versus control group, which was attenuated by (-)CBD treatment. CBD was not were able to recover the loss of adipose tissue layer induced by BLM administration (Figure 8).

Mast cells degranulation as well as macrophages and lymphocyte accumulation are the hallmark of BLM-induced fibrosis and play important roles during the inflammatory phase of SSc. Mast cells degranulation can be identified by toluidine blue staining and macrophage accumulation by staining with a mAb that identifies F4/80⁺ cells. Skin sections (5 µM-thick) were stained toluidine blue. For immunohistochemical detection of macrophages F4/80 (1:50; MCA497, Bio-Rad) antibody was used. Slides were developed with diaminobenzidine chromogen (Merck Millipore) and counterstained in Harris hematoxylin. Slides were photographed, digitalized using a Leica DFC420c camera and analyzed using Image J software. Toluidine blue staining showed an increased number of degranulated mast cells in the lesioned skin of the BLM group that was prevented by the treatment with (-)CBD (Figure 9). Moreover, immunohistochemical examination demonstrated that (-)CBD significantly reduced the infiltration of F4/80⁺ macrophages compared with BLM untreated mice (Figure 10).

### Example 6. Expression of cytokines related with inflammatory and fibrotic processes by Real-Time Quantitative PCR

Total RNA was isolated from mice frozen skin tissue using QIAzol lysis reagent (Qiagen, Hilden, Germany) and purified with RNeasy mini kit (Qiagen). Total RNA (1 µg) was retrotranscribed using the iScript™ cDNA Synthesis Kit (Bio-Rad), and the cDNA generated was analysed by real-time PCR using the iQTM SYBR Green Supermix (Bio-Rad) using a CFX96 Real-Time PCR Detection System (Bio-Rad). Real-time PCR was performed using a CFX96 Real-Time PCR Detection System (Bio-Rad). The GAPDH housekeeping gene was used to standardize the mRNA expression levels in every sample. Expression levels were calculated using the 2^{-ΔΔCt} method. Sequences of oligonucleotide primers are given in Table 1. Gene expression were normalized to GADPH mRNA levels in each sample and expressed using the 2^{-ΔΔCt} method.

**Table 1. List of mouse primer sequences used in quantitative Polymerase Chain Reaction**

| **Gene** | **Forward sequence (SEQ ID NO)** | **Reverse sequence (SEQ ID NO)** |
|---|---|---|
| IL-6 | 1 | 2 |
| IL-13 | 3 | 4 |
| Ccl2 | 5 | 6 |
| IL-1β | 7 | 8 |
| TGFβ | 9 | 10 |
| IL-4 | 11 | 12 |
| GAPDH | 13 | 14 |

The present results substantiate the therapeutic use of the CBD isomers described in the present inventions, for the clinical management of fibrotic diseases.

The expression of cytokines related with inflammatory and fibrotic processes was studied. As expected, BLM-treated mice had significantly higher expression levels of *Il13, IL1β, IL6, CCL2, TGFβ* and *IL4* in the skin compared with control mice. Such upregulation was significantly reduced in mice treated either with (-)CBD (Figure 11).

### Sequence Listing Free Text

**Table 2. Sequence Listing Free Text**

| Sequence (SEQ ID NO) | Sequence Listing Free Text | Sequence (SEQ ID NO) | Sequence Listing Free Text |
|---|---|---|---|
| 1 | IL-6 forward primer | 2 | IL-6 reverse primer |
| 3 | IL-13 forward primer | 4 | IL-13 reverse primer |
| 5 | Ccl2 forward primer | 6 | Ccl2 reverse primer |
| 7 | IL-1 β forward primer | 8 | IL-1 β reverse primer |
| 9 | TGFβ forward primer | 10 | TGFβ reverse primer |
| 11 | IL-4 forward primer | 12 | IL-4 reverse primer |
| 13 | GAPDH forward primer | 14 | GAPDH reverse primer |

## Claims

1. A compound or a pharmaceutical composition comprising said compound and at least a pharmaceutically acceptable excipient or carrier, for use in the treatment and/or prevention of fibrotic diseases and/or in the treatment and/or prevention of fibrotic symptoms of fibrotic diseases in an animal, **characterized in that** said compound or pharmaceutical composition is topically administered to the animal, wherein said compound is selected from the group consisting of a compound of Formula Ia or Ib 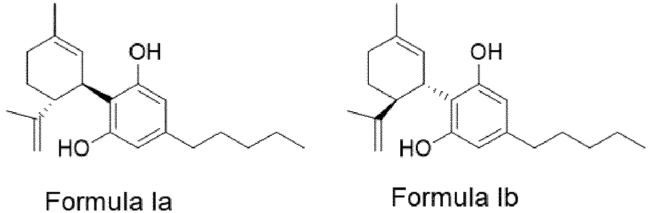 and any pharmaceutically acceptable salt or solvate thereof and wherein said fibrotic diseases and/or fibrotic symptoms of fibrotic diseases are selected from the group consisting of scleroderma, eosinophilic fasciitis, lupus, discoid lesions associated with lupus, discoid lesions associated with surgical adhesions, mixed connective tissue disease, fibrodysplasia, fibrocystic disease, sarcoidosis, myositis, inclusion body myositis, polymyositis, primary idiopathic polymyositis, childhood polymyositis, dermatomyositis, childhood dermatomyositis, primary idiopathic dermatomyositis in adults, carpal tunnel syndrome, Dupuytren's contracture, Limited Joint Mobility, vasculitis, coronary artery vasculitis, polyarteritis nodosa, temporal arteritis, cerebrosclerosis, annular sclerosis, diffuse sclerosis and lobar sclerosis.

2. Compound or pharmaceutical composition for use, according to claim 1, **characterized in that** said animal is a human.

3. Compound or pharmaceutical composition for use, according to claim 1 or 2, **characterized in that** said compound is combined with, or said pharmaceutical composition comprises, at least an antioxidant.

4. Compound or pharmaceutical composition for use, according to claim 3, **characterized in that** said antioxidant is selected from the group consisting of vitamin A, vitamin B6, vitamin C, vitamin E, glutathione, β-carotene, α-lipoic acid, coenzyme Q10, selenium and zinc.

5. Compound or pharmaceutical composition for use, according to claim 1 or 2, **characterized in that** said compound is combined with, or said pharmaceutical composition comprises, a PPARγ agonist.

6. Compound or pharmaceutical composition for use, according to claim 1 or 2, **characterized in that** said compound is combined with, or said pharmaceutical composition comprises, at least an immunosuppressive drug, Vitamin D3, or a histone deacetylase inhibitor (HDACi), or combinations thereof.

7. Compound or pharmaceutical composition for use, according to any one of claims 1-3, **characterized in that** the treatment with said compound or pharmaceutical composition is combined with a treatment with a PPARγ agonist, an immunosuppressive drug, vitamin D3, a histone deacetylase inhibitor (HDACi), or combinations thereof.

8. A cosmetic composition comprising a compound selected from the group consisting of a compound of Formula Ia or Ib 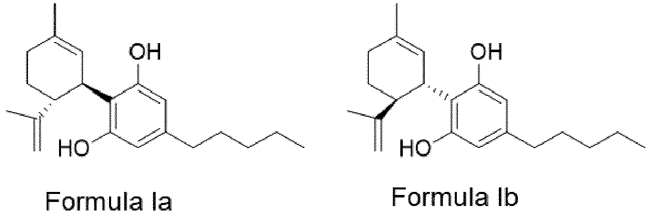 and any cosmetically acceptable salt or solvate thereof, and at least a cosmetically acceptable excipient or carrier, wherein said cosmetic composition is topically administered to the skin of a subject for preventing keloids, increasing skin elasticity and/or reducing skin itching and/or reducing skin pain in said subject.

9. Use of a compound or a cosmetic composition comprising said compound and at least a cosmetically acceptable excipient or carrier, wherein said compound or cosmetic composition is topically administered to the skin of a subject for preventing keloids, increasing skin elasticity and/or reducing skin itching and/or reducing skin pain in said subject, and wherein said compound is selected from the group consisting of a compound of Formula Ia or Ib 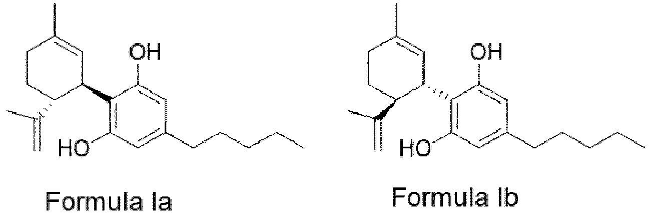 and any cosmetically acceptable salt or solvate thereof.
